(19) Europäisches Patentamt
European Patent Office
Office européen des brevets

(11) EP 2 196 224 A1

# (12) EUROPEAN PATENT APPLICATION

(43) Date of publication:
**16.06.2010 Bulletin 2010/24**

(21) Application number: **08171355.4**

(22) Date of filing: **11.12.2008**

(51) Int Cl.:
***A61L 15/28*** *(2006.01)* ***C08B 11/04*** *(2006.01)*
***C08B 11/10*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Speciality Fibres and Materials Limited**
**5 Montague Close**
**London SE1 9BB (GB)**

(72) Inventor: **The designation of the inventor has not yet been filed**

(74) Representative: **Adamson Jones**
**BioCity Nottingham**
**Pennyfoot Street**
**Nottingham NG1 1GF (GB)**

## (54) Absorbent material

(57) This invention relates to absorbent materials useful in the manufacture of absorbent articles, in particular dressings for the advanced woundcare market. The absorbent materials of the present invention are sulfonated polysaccharides, particularly water-insoluble cellulose alkyl sulfonates in which the cellulose is substituted by one type of alkyl sulfonate group. The invention also provides a process for the manufacture of such materials. The preferred cellulose alkyl sulfonate described herein is cellulose ethyl sulfonate.

Figure 1

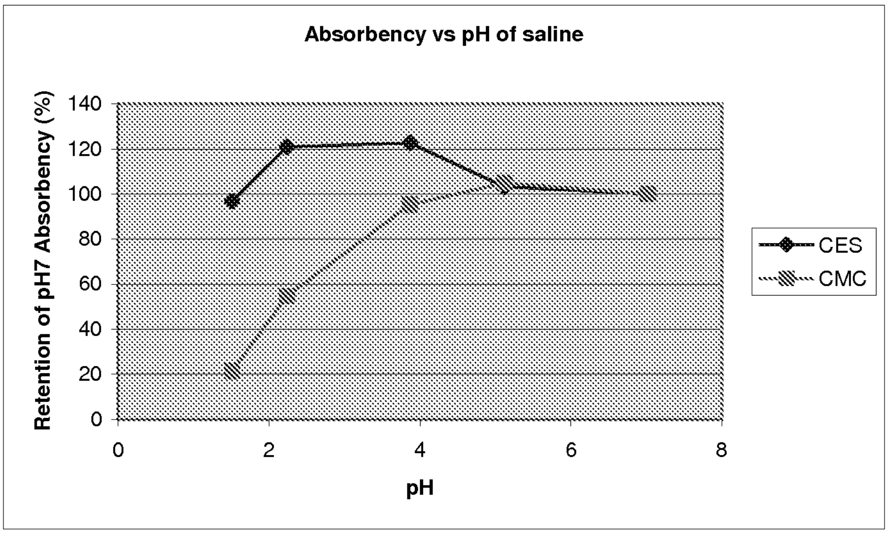


EP 2 196 224 A1

## Description

This invention relates to absorbent materials useful in the manufacture of absorbent articles, in particular dressings for the advanced woundcare market. The absorbent materials of the present invention are sulfonated polysaccharides, particularly water-insoluble cellulose alkyl sulfonates in which the cellulose is substituted by one type of alkyl sulfonate group. The invention also provides a process for the manufacture of such materials. The preferred cellulose alkyl sulfonate described herein is cellulose ethyl sulfonate.

### Background

Absorbent fibres useful as components in advanced woundcare dressings are known in the art, particularly fibres based on alginic acid, carboxymethylcellulose and carboxymethylchitosan, and salts thereof.

Dressings based on fibres of alginic acid or its salts have good overall absorbency of wound fluid, but suffer from slow absorption due to the need to exchange multivalent ions binding the fibrous structure together with sodium ions present in wound fluid. Although this ion exchange renders the fibres swellable in ion-containing aqueous media, allowing significant absorption of fluid, the mechanical strength of the gelled fibres is compromised, and it is not routinely possible to remove a saturated dressing in one piece. Frequently, the dressing must be irrigated with saline to wash it away, and this can be traumatic for the patient.

Carboxymethyl cellulose fibres have also been used as the main component in advanced woundcare dressings, and these too have significant absorptive capacity for wound fluid. Their advantage over alginate-type dressings is that absorption of fluid is virtually instantaneous since no ionic exchange is required to render the fibres gellable. In addition, those fibres based on a highly crystalline cellulose, such as lyocell, and in particular those described in EP0616650 and EP0680344, tend to retain a higher level of mechanical strength and therefore may be removed from the wound site in one piece. However the absorptive capacity of this class of material is strongly dependent on the pH of the wound fluid, reducing dramatically at acidic pH. This is a serious drawback since chronic wound fluid pH can range between 4 and 8 depending on the state of healing. Furthermore, it has been recognised that artificially lowering the pH of the wound environment may lead to improved healing outcomes. For instance, it has been found (Tsioras et al, article presented at 19th Annual Symposium on Advanced Wound Care, San Antonio, TX, 30 April 2006 - 3 May 2006) that applying a wound dressing containing a pH adjusting cream of pH 2.8 decreased the time it took for the wound to close. In another study, burn wounds healed quicker when treated with fluid having a pH of 3.5 (Kaufman T et al, Burns Incl Therm Inj, 1985, 12(2) 84-90). Indeed, preparations are commercially available for use in conjunction with absorbent dressings to reduce the pH of the wound environment. For instance, Cadesorb® available from Smith & Nephew has a pH of about 4.35.

It is desirable for an absorbent dressing to perform well at acidic pH, and preferably for it to perform well over a wide range of pH. Since absorbent dressings based on carboxymethylcellulose do not perform well in low pH environments, there is a need for an instantly gelling, absorptive dressing that continues to absorb to a good level at reduced pH.

It is desirable for absorbent fibres for use in absorbent dressings to be obtained from a renewable resource, to be inexpensive and also biodegradable. Hence, there is considerable interest in cellulose as a renewable and biodegradable source of absorbent material. US southern pine fluff pulp is used as an absorbent material in the personal care industry. However, it is commonly used in conjunction with other absorbent materials, and commonly materials that are not renewable and biodegradable, for example acrylic acid polymers. The reason for this is that absorbed liquid is not effectively retained in materials that are made exclusively of cellulosic fibres.

The cellulose fibre can be modified by sulfonation, for example by substitution with an alkyl sulfonate at one or more of the hydroxyl groups on the anhydroglucose monomers that make up the cellulose backbone, forming ether linkages. Cellulose derivatives of this type are known as cellulose sulphonates or cellulose alkyl sulphonates.

Commercially available cellulose ethers are, as a rule, water-soluble compounds. In particular, cellulose ethyl sulfonate is known to be water-soluble.

US4990609 describes cellulose ethyl sulfonates of high solution quality, which are prepared by addition to cellulose of an alkylating agent and subsequently addition of alkali. The process is compared to the two-stage process for the production of cellulose ethyl sulfonate described in SU757540.

Cellulose ether sulfonates have been modified further in order to produce water-insoluble products. For instance US2006/0142560 refers to absorbent fibres based on mixed cellulose alkylsulphonates in which the cellulose is substituted by two different groups, an alkyl sulphonate and a hydroxyalkyl sulphonate, specifically ethyl sulfonate and 2-hydroxypropyl sulfonate. Water-insolubility of the modified cellulose is believed to result from the presence of the 2-hydroxypropyl sulfonate group.

US5703225 refers to a water-insoluble sulfonated cellulose that is a hydroxy sulfonic cellulose in which both the sulphur atom of a sulfonic group and a hydroxyl group are directly attached to a carbon atom on the cellulose chain.

To be suitable for use in wound dressings, absorbent materials must retain their integrity and hence be water-insoluble. The principal disadvantage of the water-insoluble cellulose alkyl sulfonates that have been developed for use as absorbent materials to date is the requirement for substitution of the cellulose with at least two different groups. Compared to substitution with a single substituent, additional reactants and additional processing steps are not desirable, and are likely to increase the cost of manufacture. Furthermore, as the cellulose is increasingly modified, benefits associated with the natural fibre, such as its biodegradability, may be impaired.

## Summary of the Invention

It has surprisingly been found that water-insoluble cellulose alkyl sulfonates may be prepared by the substitution of cellulose with only one type of alkyl sulfonate.

It will be clear to those practised in the art that other polysaccharide substrates could be converted to an alkyl sulfonate derivative in accordance with the invention. For example, chitin and chitosan are natural polysaccharides based on D-glucosamine units, which have hydroxyl groups at positions at C3 and C5 where reaction substitution with alkyl sulphonate groups can take place. In addition, it is possible to substitute at the amine group in the C2 position, attaching the alkyl sulfonate via the nitrogen.

Thus, according to a first aspect of the invention there is provided an absorbent article comprising, as an absorbent material, a water-insoluble polysaccharide alkyl sulfonate, wherein the polysaccharide is substituted with one type of alkyl sulfonate.

The modified polysaccharides of the invention are highly advantageous for use as absorbent materials in wound dressings because they exhibit excellent absorption and retention of fluid whilst maintaining their integrity sufficiently to be removed from the wound site in one piece, without irrigation, and with minimum pain and shedding. As with carboxymethyl cellulose, absorption of fluid is virtually instantaneous since ionic exchange is not required for the fibres to become gellable. However, the water-soluble polysaccharide alkyl sulfonates of the present invention are advantageous compared to carboxymethyl cellulose because the absorptive capacity may be affected to a lesser extent by changes in pH. Wound dressings containing these materials may continue to absorb to a good level at low pH.

The polysaccharide alkyl sulfonate may be used in the form of fibres.

The polysaccharide alkyl sulfonate may be a cellulose alkyl sulfonate, and the following description refers primarily to such embodiments of the invention. It will be appreciated, however, that other polysaccharides may be utilised.

The alkyl moiety of the alkyl sulfonate substituent group is preferably a lower alkyl having 2 to 6 carbon atoms, preferably ethyl, propyl or butyl. The alkyl moiety may be branched or unbranched, and hence suitable propyl sulfonate substituents may be 1- or 2-methyl-ethylsulfonate. Butyl sulfonate substituents may be 2-ethyl-ethylsulfonate, 2,2-dimethyl-ethylsulfonate or 1,2-dimethyl-ethylsulfonate. The alkyl sulfonate substituent group that is most preferred is ethyl sulfonate.

Thus, a preferred cellulose alkyl sulfonate of the present invention is cellulose ethyl sulfonate, where ethyl sulfonate or one of its salts is attached via one or more of the hydroxyl groups on the anhydroglucose units of the cellulose. The structure of one anhydroglucose unit substituted by one ethyl sulfonate group is depicted by formula (I)

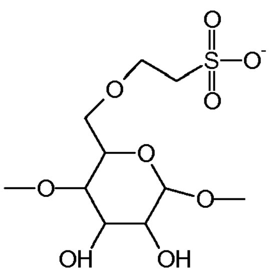

(I)

Formula (I) is not meant to depict the exact chemical structure of cellulose ethyl sulfonate prepared in accordance with the invention, because substitution can take place at any of the hydroxyl positions in the cellulose macromolecule, in any distribution up to the maximum degree of substitution that is possible.

The average degree of substitution refers to the mean number of hydroxyl positions substituted with an alkyl sulfonate substituent group, or put another way, the mean number of moles of alkyl sulfonate groups per mole of anhydroglucose unit in the cellulose polymer. The maximum degree of substitution is therefore 3, when the anhydroglucose unit is substituted at all three hydroxyl positions. The degree of substitution when an average of one hydroxyl group

is substituted per anhydroglucose unit, as shown in Formula (I), is 1.

Cellulose ethyl sulfonate is described in the prior art as a water-soluble compound, and is modified further to render it water-insoluble, eg by alkylation with 3-chloro-2-hydroxypropyl sulfonate. Water-insoluble cellulose ethyl sulfonate and other insoluble cellulose alkyl sulfonates wherein the cellulose is substituted with one type of alkyl sulfonate are therefore believed to be novel.

Thus, in a second aspect of the invention there is provided a water-insoluble cellulose alkyl sulfonate, wherein the cellulose is substituted with one type of alkyl sulfonate.

The functional properties of the cellulose alkyl sulfonates of the present invention depend on the degree of substitution, the chain length of the cellulose backbone structure and the structure of the alkyl sulfonate substituent. Solubility and absorbency are largely dependent on the degree of substitution: as the degree of substitution is increased, the cellulose alkyl sulfonate becomes increasingly soluble. It follows that, as solubility increases, absorbency increases.

To be useful in an absorbent wound dressing, absorbent materials preferably have an absorbency of at least 8 grams per gram (g/g) of 0.9% saline solution, as measured by the method described below in Example 1. Preferred cellulose alkyl sulfonates of the present invention have an absorbency (of 0.9% saline solution) of at least 8g/g, more preferably at least 9g/g, most preferably at least 10g/g.

It has been found that the average degree of substitution should preferably be less than 0.4 for the cellulose alkyl sulfonate to be substantially water-insoluble. By "substantially" is meant in this context that when the cellulose alkyl sulfonate is exposed to an excess of an aqueous medium it does not dissolve into solution, or at least that dissolution is so low as to have no significant effect on the properties of the polymer.

The average degree of substitution is preferably less than 0.4, more preferably less than 0.3. In some preferred embodiments of the invention, the average degree of substitution of the cellulose alkyl sulfonate is from about 0.05 to about 0.4, more preferably from about 0.1 to about 0.3.

Cellulose alkyl sulfonates according to the present invention can be formed by reaction of cellulose with an alkenyl sulfonate or one of its salts in the presence of a base, preferably an alkali metal hydroxide, either in aqueous or non-aqueous medium.

Alkalisation and alkyl sulfonation (which in this case is an etherification step) may be carried out as a single step in which the base and alkenyl sulfonate are added at the same time in one reaction vessel (a "one-pot" process). Alternatively, alkalisation and alkyl sulfonation may be carried out in two separate reaction steps, treating the cellulose first with alkali and then alkyl sulfonating agent, or with alkyl sulfonating agent and then alkali.

One-pot processes are often desirable because they can be easier, quicker, and by minimising the number of reaction steps a higher yield may be obtained.

When the alkali and alkyl sulfonating agent are used simultaneously in a one-pot process, to produce a cellulose alkyl sulfonate of the present invention, the reaction rate is higher than that observed for the equivalent reaction in which alkalisation and alkyl sulfonation are carried out in separate steps. As mentioned above, the greater the degree of substitution, the greater the absorbency of the cellulose alkyl sulfonate material. Thus, the reaction rate may be determined by measuring the time taken for the alkyl sulfonation reaction to yield a product having a particular degree of absorbency. In practice, it is not easy to stop a reaction at a specific absorbency level. Nevertheless, it is clear that a reaction taking 90 minutes to reach an absorbency of 14.2g/g is significantly faster than one that takes 120 minutes to reach an absorbency of only 9.7g/g.

The amount of water in the reaction mixture is also shown to affect the reaction rate. Lowering the water content in a reaction in which alkalisation and alkyl sulfonation are carried out simultaneously results in a significant increase in reaction rate. Lowering the water content in the alkyl sulfonation step of a reaction in which alkalisation and alkyl sulfonation are carried out separately increases the rate, but to a lesser extent.

A one-pot process would also be expected to minimise exposure of the cellulose to the base, therefore keeping the alkaline, oxidative degeneration of the cellulose to a minimum. It is necessary to minimise degeneration of the cellulose during processing in order to ensure that the modified cellulose is sufficiently strong to be useful as an absorbent material in a wound dressing, and indeed to maximise both the dry strength and the wet strength of the product.

However, it has been found that the strength of the fibres prepared by a one-pot process may be surprisingly and significantly weaker than fibres prepared by carrying out alkalisation and alkyl sulfonation in separate steps, depending on the level of water used in the reaction mixture.

When higher levels of water are used in the reaction, the cellulose alkyl sulfonates produced by a one-pot process have a surprisingly low fibre strength compared to the cellulose alkyl sulfonates produced by the analogous two-step process. The fibres are too weak to be suitable for processing using normal non-woven textile processing methods. If the level of water used in the reaction is reduced, the reaction rate increases and also the fibre strength increases to a useable level. According to a further aspect of the invention, there is provided a process for the preparation of water-insoluble cellulose alkyl sulfonate comprising the simultaneous reaction of cellulose with an alkali and alkyl sulfonating agent, and wherein the weight of water present in the reaction is less than 950% of the (dry) weight of the cellulose.

According to a yet further aspect of the invention, there is provided a process for the preparation of a water-insoluble cellulose alkyl sulfonate, which process comprises the separate steps of:

a) treating the cellulose with alkali;
b) reacting the product of step a) with an alkenyl sulfonate or its salt; and
c) isolating the product of step b).

This two-step process is surprisingly beneficial when the level of water used in step b) is more than 950% of the (dry) weight of the cellulose.

Detailed Description of the Invention

Processes for preparing the cellulose alkyl sulfonates of the present invention were compared using 47% NaOH solution, 25% sodium vinylsulfonate solution, and different quantities of water in the alkyl sulfonation reaction. The properties of the fibres are presented in Table 1 below:

Table 1

| Reaction type | Time of alkylsulfonation (min) | Weight water in alkylsulfonation (% of fibre wt) | Weight of NaOH in alkylsulfonation (% fibre wt) | Absorbency (g/g) | Filament strength |
|---|---|---|---|---|---|
| Two-step high water (SFM006/69) | 120 | 970 | 197 | 9.7 | OK |
| Two-step low water (SFM006/136) | 120 | 460 | 177 | 13.7 | OK |
| One-pot high water (SFM006/148c) | 90 | 960 | 198 | 14.2 | Low |
| One-pot low water (SFM006/145a) | 70 | 630 | 198 | 11.9 | OK |

Comparable amounts of water were used in the two-step high water reaction and the one-pot high water reaction. The reaction rate of the one-pot process was significantly higher, but the filament strength of the cellulose ethyl sulfonate product produced by the one-pot process was lower than the strength of the fibre produced by the two-step process with separate alkalisation and alkyl sulfonation steps. Indeed, the filament strength of the product of the one-pot process was too low for the material to be effective for use, as intended, in wound dressing applications.

In the one-pot process for the preparation of water-insoluble cellulose alkyl sulfonate comprising the simultaneous reaction of cellulose with an alkali and alkyl sulfonating agent, the weight of water present in the reaction is less than 950% of the (dry) weight of the cellulose, more preferably less than 800% of the weight of the cellulose, more preferably less than 700% by weight of the cellulose, and most preferably less than 650% by weight of the cellulose.

Reducing the level of water in the second alkyl sulfonation step in the two-step process is shown to increase the reaction rate. However, it is not always practicable to carry out that reaction step using lower levels of water, as it becomes increasingly difficult to wet the cellulose as the volume of alkyl sulfonate reactant decreases. In any case, when using lower levels of water in the reaction, the one-pot process is preferred.

At higher levels of water, the two-step process is most suitable for producing cellulose alkyl sulfonates of the present invention with adequate fibre strength. Preferably the weight of water present in the alkyl sulfonation step is more than 650% by (dry) weight of the cellulose, more preferably more than 750% by weight of the cellulose, more preferably more than 850% by weight of the cellulose, and most preferably more than 950% by weight of the cellulose.

To be suitable for use in the present invention, the cellulose is preferably fibrous in nature. The cellulose fibres should have a high degree of crystallinity and total orientation in order that the fibres maintain sufficient strength after derivatisation to be processed, and that the resulting material is strong enough for its intended use.

In particular, the use of alkali in the alkalisation step can degrade the cellulose backbone, causing chain scission

and a reduction in the degree of polymerisation, thereby resulting in the fibres having a lower strength after derivatisation. The dry strength of the derivatised fibres must be sufficient to enable processing into woven or nonwoven structures, and, to be useful as an absorbent material in a wound dressing, the wet strength of the material must be sufficient to allow removal from the site in one piece.

Fibrous celluloses with a high degree of crystallinity, that are particularly suitable for use in the invention, include cotton or regenerated cellulose fibres such as lyocell.

It will be clear to those skilled in the art that it is possible to sulphonate particulate cellulose such as pulp fibres, then dissolve the sulphonated cellulose in a suitable solvent, such as a lyocell solvent or an ionic liquid, and spin the sulphonated cellulose as fibres, or extrude the sulphonated cellulose as a film or other extrusion to produce the absorbent material of the invention. Furthermore, a blowing agent could be added to the solution in order to produce a foamed absorbent material.

The cellulose may be alkalised by treatment with a strong alkali, preferably an alkali metal hydroxide such as sodium hydroxide. 47% sodium hydroxide solution has been found to be suitable. Generally, the higher the concentration of alkali and the higher the reaction temperature, the faster the rate of reaction. The strength of the reaction conditions should be balanced with the need to avoid degradation of the cellulose substrate. However, the level of degradation of the cellulose is considerably lower than might be expected under the relatively intense reaction conditions that are required for alkalisation. When carrying out the two-step process, it may be beneficial to remove excess alkali before proceeding with second alkyl sulfonation step, eg by mechanically squeezing the alkalised fibres.

The alkyl sulfonation step (or etherification step) is the nucleophilic addition of the alkoxide ion to an alkenyl sulfonate, specifically $\alpha$-alkenyl sulfonate or its salt. The $\alpha$-alkenyl sulfonate is preferably a lower alkenyl sulfonate, in which the alkenyl moiety has 2 to 6 carbon atoms. Preferably the $\alpha$-alkenyl sulfonate is vinyl sulfonate, allyl sulfonate (1-propenyl sulfonate), isopropenyl sulfonate (1-methylvinyl sulfonate), 1-butenyl sulfonate, 1-methyl allyl sulfonate (1-methyl-1-propenyl sulfonate) or 2-methylallyl sulfonate (2-methyl-1-propenyl sulfonate). In a particularly preferred embodiment, the $\alpha$-alkenyl sulfonate is vinyl sulfonate, more preferably the sodium salt of vinyl sulfonate, and hence the cellulose alkyl sulfonate product is cellulose ethyl sulfonate.

The sodium salt of vinyl sulfonate is commercially available as an approximately 30% aqueous solution. It may be brought into contact with the cellulose or alkalised cellulose by methods known in the art, for instance spraying onto the cellulose, or mixing using stirrers. The conversion to cellulose alkyl sulphonate can take place at any temperature up to the boiling point of the reaction mixture, or beyond it if a pressurised system is used. Obviously, the rate of reaction is increased if the reaction stage is carried out at elevated temperature. The preferred range is 30-95°C to give a useful degree of substitution in an economic time. Further, fresh charges of reactant can be introduced at any time throughout the reaction. The degree of substitution can be controlled by control of reaction temperature and, in particular, by control of the reaction time.

Vinyl sulfonate is thought to be less harzardous than some of the halogenated reactants, particularly chlorinated reactants, which are typically used to prepare the absorbent materials currently available for use in woundcare products. Certainly, chloroacetic acid, used in the manufacture of carboxymethyl cellulose, is a potentially dangerous alkylating agent. Its use during the manufacturing process is undesirable, and retention of any residual chloroacetic acid in the absorbent product may be harmful, at the least causing skin irritation. The use of only one type of alkyl sulfonate also presents potential advantages in terms of safety and removal of residual reactant compared to other water-insoluble cellulose alkyl sulfonates that are known, in which the cellulose is substituted with more than one type of alkyl sulfonate, if only because of the relative simplicity of the chemistry.

After the reaction has proceeded to the desired extent, the reaction can be stopped by neutralising the reaction mixture, ie reducing the pH to approximately neutral by addition of acid. The acid may be any common mineral or organic acid such as hydrochloric or acetic acid respectively. The cellulose alkyl sulfonate product can then be washed free of by-products and impurities by employing washing stages known in the art. Such stages include washing with water, organic liquids or mixtures thereof. Particularly useful are mixtures of a lower alcohol and water. Washing efficiency can be enhanced by washing at elevated temperature. After washing, it may be desirable to apply a processing aid, such as glycerol, as is common practice in the production of, for example, cellulose film (cellophane). This can be achieved by methods known in the art, such as dipping, spraying etc.

Finally the derivatised cellulose article should be dried to remove residual liquid from the previous stages. Drying can be carried out by methods known in the art such as forced air drying, radiant heat drying etc.

The absorbent materials of the present invention exhibit instant gelling in aqueous media, good absorbency and, crucially, good retention of absorbency in an acidic environment. This renders them ideal for use as an absorbent wound dressing, or as part of an absorbent dressing. They are particularly useful for wounds with moderate to high levels of exudates, and for flat or cavity wounds of this type. Typical examples include pressure sores and leg ulcers.

The use of the absorbent materials of the present invention is not limited to woundcare products, indeed they are expected to be useful for many other applications. Their absorbent properties, biodegradability, and the fact that cellulose is a renewable material, mean that the cellulose alkyl sulfonates of the invention are also particularly desirable

for use in the personal care sector, particularly for disposable sanitary articles such as nappies (diapers), disposable nappies and training pants, feminine care products, eg tampons, sanitary towels or napkins and pant liners, and incontinence products. The simplicity of the chemistry and the availability of the reactants enable the cost of manufacture of such articles to be kept advantageously low.

Other medical products are envisaged, for example surgical and dental sponges, and the materials could also be useful in packaging, for example as absorbent pads in food containers.

The cellulose alkyl sulfonates of the present invention may be processed according to known methods into a wide variety of forms, depending on their intended use. The manner in which the derivatised cellulose is processed has a significant effect on the properties of the final product, particularly the strength, gelling time and absorbency. Preferred cellulose alkyl sulfonate products for use in woundcare articles are carded, needle-bonded nonwovens.

The invention will now be illustrated by the following non-limiting examples.

Example 1 - Method for determination of the free absorbency of fibres

The fibre was cut into a 2-3mm flock and 0.5g of cut fibre was placed in a 100ml screw-top jar. 50ml of test liquid (eg 0.9% saline, typically used to simulate the ionic strength of wound fluid) was added and the jar shaken for 30 seconds to disperse the flock. The dispersion was then filtered through a 47mm Buchner funnel fitted with a 42.5mm diameter Whatman No. 4 filter paper, using a vacuum pump, with vacuum set to be greater than 0.8 bar for 1 minute. Then the fibre dispersion was removed and weighed. Free absorbency was calculated using following formula:

$$absorbency(g/g) = \left[\frac{wet_dispersion_weight(g)}{dry_flock_weight(g)}\right] - 1$$

Example 2 - Method for determination of breaking tenacity and elongation of single filaments

Tenacity and elongation at break of dry, single filaments was carried out using a tensile testing machine, fitted with appropriate jaws for gripping single filaments and load cell of the appropriate range.

The samples were conditioned for at least four hours and were tested in the standard atmosphere for testing textiles (20 ± 2°C and 65 ± 2% relative humidity). The machine was balanced and calibrated according to the manufacturer's instructions. Filaments were taken at random from different parts of the sample. The linear density of the filament was measured by an appropriate technique such the Vibraskop method. The filament was then placed between the jaws of the tensile testing machine and the test started. The following conditions were used:

| | |
|---|---|
| Test length: | 20 mm |
| Load range: | 0-10 cN |
| Cross-head speed: | 10 mm/minute |
| Chart speed (when applicable): | 10 -20 mm/minute |
| Number of tests: | 10 |

After rupture, the crosshead was returned and the broken ends of filament were checked and removed from the jaws. A note was made if the number of jaw breaks exceeded 10%.

The breaking load (cN) and the breaking extension (%) of each filament was usually printed out together with the statistics. In the case of individual breaking loads being printed out, individual tenacity results or a mean tenacity were calculated by hand, as follows:

$$\text{mean tenacity (cN/tex)} = \frac{\text{mean breaking load in cN x 10}}{\text{mean linear density in dtex}}$$

Example 3 - Preparation of cellulose ethyl sulfonate using a two-step process

A 3g sample of lyocell tow, known under the tradename Tencel® (manufactured by Lenzing), was immersed

in aqueous 47% sodium hydroxide for 25 minutes at 25°C. Excess sodium hydroxide was then removed by squeezing. Then 25ml 30% sodium vinyl sulphonate solution (Fluka Chemicals) was added to the fibre and heated at 91 °C for 90 minutes. After this time the reaction mixture was neutralised to pH 7 by adding glacial acetic acid dropwise. Then the excess liquid was squeezed from the fibre and the fibre washed twice in a mixture of industrial methylated spirit (IMS) and water (80:20 v/v). After drying to constant weight at 60°C, the fibre was tested for absorbency.

Using the method outlined in Example 1, and an aqueous solution of 0.9% NaCl as the test liquid, an absorbency value of 11.1 g/g was achieved.

Example 4 - Preparation of cellulose ethyl sulfonate using a two-step process (SFM006/69)

A 2.5g sample of Tencel® fibre was immersed in 47% sodium hydroxide for 30 minutes at 20°C, after which excess liquor was removed by squeezing. 21 ml sodium vinyl sulphonate (30% aqueous solution) was poured over the fibre. The vessel containing fibre and reactant was then heated at 83°C for 2 hours, after which time the sample was neutralised by the dropwise addition of glacial acetic acid until a pH of 7 was reached. The excess liquor was then squeezed from the fibre, and the fibre washed twice with a IMS/water (80:20 v/v), and finally in 100% IMS. After drying to constant weight at 60°C, the fibre was tested for absorbency according to Method 1, using 0.9% aqueous NaCl as the test liquid. A value of 9.7g/g was obtained.

Example 5 - Preparation of cellulose ethyl sulfonate using a one-pot process with high water content

3g Tencel® fibre was immersed in a mixture of 10ml 47% NaOH and 25ml 30% sodium vinylsulphonate solution, and heated for 75 minutes at 83°C. The reaction mixture was then neutralised by addition of acetic acid, after which the fibre was removed and washed in a mixture of IMS/water (80:20 v/v), and finally in 100% IMS. Drying was carried out at 60°C.

Using the method outlined in Example 1, and an aqueous solution of 0.9% NaCl as the absorbent test liquid, an absorbency value of 6.6g/g was achieved. The fibres were visibly weaker than the fibres of Example 6, despite having a lower degree of substitution, as evidenced by the lower absorbency value.

Example 6 - Preparation of cellulose ethyl sulfonate using a one-pot process with low water content (SFM006/145a)

3g Tencel® fibre was immersed in a mixture of 13ml 30% sodium vinylsulphonate solution and 10ml 47% NaOH solution, and heated for 70 minutes at 83°C. The reaction mixture was then neutralised by addition of acetic acid, after which the fibre was removed and washed in a mixture of IMS/water (80:20 v/v), and finally in 100% IMS. Drying was carried out at 60°C.

Using the method outlined in Example 1, and an aqueous solution of 0.9% NaCl as the test liquid, an absorbency value of 11.9g/g was achieved.

Example 7 - Comparative absorbency test for underivatised cellulose

Tencel® fibre from the same batch used as the starting material for Examples 3 and 4 was subjected to the absorbency test outlined in Example 1, using 0.9% aqueous NaCl as the absorbent test liquid. A value of 0.9g/g was obtained.

Example 8 - Comparison of the absorbency at low pH of cellulose ethyl sulfonate fibres of the present invention with carboxymethyl cellulose fibres of the prior art

The absorbency of carboxymethyl cellulose (CMC) fibres made according to the teachings in EP0616650 was measured according to the method of Example 1 using 0.9% saline solution as absorbing liquid. The pH of the saline was then reduced successively by addition of 37% HCl and the absorbency measured again at each pH.

Cellulose ethyl sulfonate fibres were produced according to the present invention from lyocell fibre and their absorbency measured in the same way at a range of pH values.

The results are shown graphically in Figure 1. It is clear that cellulose ethyl sulfonate fibre of the invention retains significantly more of its absorbency at low pH where wound healing is believed to be enhanced.

## Claims

1. An absorbent article comprising, as an absorbent material, a water-insoluble polysaccharide alkyl sulfonate, wherein

the polysaccharide is substituted with one type of alkyl sulfonate.

**2.** An article as claimed in Claim 1, wherein the polysaccharide is cellulose.

**3.** An article as claimed in Claim 1 or Claim 2, wherein the alkyl sulfonate moiety is ethyl sulfonate.

**4.** A water-insoluble cellulose alkyl sulfonate, wherein the cellulose is substituted with one type of alkyl sulfonate.

**5.** A cellulose alkyl sulfonate as claimed in Claim 4, which has an absorbency (of 0.9% saline solution) of at least 8g/g.

**6.** A cellulose alkyl sulfonate as claimed in Claim 4 or Claim 5, wherein the mean degree of substitution is less than 0.4 alkyl sulfonate groups per anhydroglucose unit.

**7.** A cellulose alkyl sulfonate as claimed in any one of Claims 4 to 6, wherein the alkyl sulfonate moiety is ethyl sulfonate.

**8.** Use of a water-insoluble cellulose alkylsulfonate as claimed in any one of Claims 4 to 7 as an absorbent material.

**9.** A process for the preparation of water-insoluble cellulose alkyl sulfonate comprising the simultaneous reaction of cellulose with an alkali and an alkenyl sulfonate or its salt, and wherein the amount of water present in the reaction is less than 950% by weight of the cellulose.

**10.** A process for the preparation of a water-insoluble cellulose alkyl sulfonate, which process comprises the separate steps of:

a) treating the cellulose with alkali;
b) reacting the product of step a) with an alkenyl sulfonate or its salt; and
c) isolating the product of step b);

**11.** A process as claimed in Claim 10, wherein the amount of water present in step b) is more than 650% by weight of the cellulose.

**12.** A process as claimed in any one of Claims 9 to 11, wherein the alkenyl sulfonate is an $\alpha$-alkenyl sulfonate.

**13.** A process as claimed in Claim 12, wherein the alkenyl sulfonate is selected from the group consisting of vinyl sulfonate, allyl sulfonate, isopropenyl sulfonate, 1-butenyl sulfonate, 1-methyl allyl sulfonate and 2-methylallyl sulfonate.

**14.** A process as claimed in Claim 13, wherein the alkenyl sulfonate is vinyl sulfonate.

**15.** A cellulose alkyl sulfonate prepared by a process as claimed in any one of Claims 9 to 14.

Figure 1

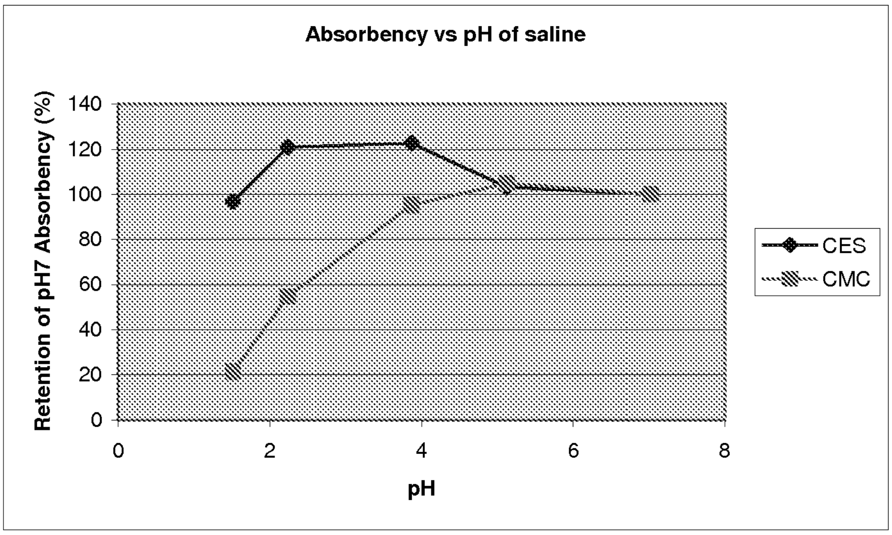
Absorbency vs pH of saline
Retention of pH7 Absorbency (%)
140
120
100
80
60
40
20
0
0
2
4
6
8
pH
CES
CMC

Europäisches Patentamt
European Patent Office
Office européen des brevets

# EUROPEAN SEARCH REPORT

Application Number
EP 08 17 1355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online] CHEMICAL ABSTRACTS SERVICE, COLUMBUS, OHIO, US; T. TIMELL: "Investigations on cellulose reactions. III. The preparation and properties of sulfoethylcellulose" XP002527652 retrieved from STN-INTERNATIONAL Database accession no. 43:1629 | 4-7,15 | INV. A61L15/28 C08B11/04 C08B11/10 |
| Y | * abstract * & SVENSK PAPPERSTIDNING, vol. 51, 1948, pages 254-258, ----- | 1-3,8-14 | |
| Y | DATABASE CA [Online] CHEMICAL ABSTRACTS SERVICE, COLUMBUS, OHIO, US; S.V.NAKHOROSHKOV ET AL.: "Explanation of the cellulose sulfoethylation mechanism" XP002527653 retrieved from STN-INTERNATIONAL Database accession no. 110:156307 * abstract * & KOKSNES KIMIJA, no. 6, 1988, pages 20-27, ----- | 8-14 | TECHNICAL FIELDS SEARCHED (IPC): A61L C08B |
| X | EP 0 210 756 A (PROCTER & GAMBLE [US]) 4 February 1987 (1987-02-04) * page 2, lines 13-19 * * page 4, lines 11-25 * * page 5, lines 1-10 * * page 6, lines 4-8 * * page 7, lines 10-14 * * page 9, line 35 - page 10, line 34 * * page 16, lines 14-29 * * claims 1,9 * ----- -/-- | 1-8,15 | |

The present search report has been drawn up for all claims

| Place of search | Date of completion of the search | Examiner |
|---|---|---|
| The Hague | 10 August 2009 | Lamers, Wolfram |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

2

Europäisches Patentamt
European Patent Office
Office européen des brevets

# EUROPEAN SEARCH REPORT

Application Number
EP 08 17 1355

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 813 900 A (MO OCH DOMSJOE AB) 27 May 1959 (1959-05-27) * page 1, lines 10-32 * * page 3, lines 10-17,114-116 * ----- | 4-6,15 | |
| X | EP 0 511 540 A (WOLFF WALSRODE AG [DE]) 4 November 1992 (1992-11-04) * page 2, lines 29,30,32-34 * * page 3, lines 32-35 * ----- | 4-7,15 | |
| X | US 2 580 352 A (GRASSIE VERNON R) 25 December 1951 (1951-12-25) * column 1, lines 1-4 * * example 6 * ----- | 4-7,10, 12-15 | |
| Y | US 2006/142484 A1 (GLASSER WOLFGANG G [US] ET AL) 29 June 2006 (2006-06-29) * paragraphs [0012], [0016] - [0021] * ----- | 1-3,8 | |
| D,Y | US 2006/142560 A1 (GLASSER WOLFGANG G [US] ET AL) 29 June 2006 (2006-06-29) * paragraphs [0005], [0006], [0012], [0047] * ----- | 1-3,8 | TECHNICAL FIELDS SEARCHED (IPC) |
| Y | US 4 783 448 A (JOHANSSON OLOF J A [SE]) 8 November 1988 (1988-11-08) * column 1, lines 26-58 * * column 1, line 64 - column 2, lines 8,59-62 * * column 3, lines 24-34,43-50 * ----- | 1-3,8 | |

The present search report has been drawn up for all claims

| Place of search | Date of completion of the search | Examiner |
|---|---|---|
| The Hague | 10 August 2009 | Lamers, Wolfram |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 17 1355

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2009

| Patent document cited in search report | | Publication date | Patent family member(s) | | | Publication date |
|---|---|---|---|---|---|---|
| EP 0210756 | A | 04-02-1987 | AT | 109984 | T | 15-09-1994 |
| | | | AU | 590581 | B2 | 09-11-1989 |
| | | | AU | 5931786 | A | 08-01-1987 |
| | | | CA | 1296875 | C | 10-03-1992 |
| | | | DE | 3650029 | D1 | 22-09-1994 |
| | | | DE | 3650029 | T2 | 02-03-1995 |
| | | | ES | 2000176 | A6 | 01-01-1988 |
| | | | GR | 861662 | A1 | 05-09-1986 |
| | | | IE | 63575 | B1 | 17-05-1995 |
| | | | JP | 2564277 | B2 | 18-12-1996 |
| | | | JP | 62079845 | A | 13-04-1987 |
| | | | PH | 25565 | A | 08-08-1991 |
| | | | US | 4818598 | A | 04-04-1989 |
| GB 813900 | A | 27-05-1959 | NONE | | | |
| EP 0511540 | A | 04-11-1992 | CA | 2067015 | A1 | 28-10-1992 |
| | | | DE | 4113892 | A1 | 29-10-1992 |
| | | | JP | 3319613 | B2 | 03-09-2002 |
| | | | JP | 5178901 | A | 20-07-1993 |
| | | | US | 5278304 | A | 11-01-1994 |
| US 2580352 | A | 25-12-1951 | NONE | | | |
| US 2006142484 | A1 | 29-06-2006 | NONE | | | |
| US 2006142560 | A1 | 29-06-2006 | NONE | | | |
| US 4783448 | A | 08-11-1988 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0616650 A
- EP 0680344 A
- US 4990609 A
- SU 757540
- US 20060142560 A
- US 5703225 A


### Non-patent literature cited in the description


- **Tsioras et al.** *19th Annual Symposium on Advanced Wound Care,* 30 April 2006
- **Kaufman T et al.** *Burns Incl Therm Inj,* 1985, vol. 12 (2), 84-90